Europäisches Patentamt

European Patent Office

Office européen des brevets

(19)

(11) Publication number: **0 319 307**
**A2**

(12)

# EUROPEAN PATENT APPLICATION

(21) Application number: **88311408.4**

(22) Date of filing: **01.12.88**

(51) Int. Cl.4: **C 12 N 15/00**
**A 61 K 37/02, G 01 N 33/566**

(30) Priority: **04.12.87 US 129002**

(43) Date of publication of application:
**07.06.89 Bulletin 89/23**

(84) Designated Contracting States: **ES GR**

(71) Applicant: **Schering Biotech Corporation**
**901 California Avenue**
**Palo Alto California 94304-1104 (US)**

(72) Inventor: **Moore, Kevin W.**
**4144 Park Blvd.**
**Palo Alto California 94306 (US)**

**Peltz, Gary A.**
**1145 Divisadero Street Apt. No. 3**
**San Francisco California 94115 (US)**

(74) Representative: **Ritter, Stephen David et al**
**Mathys & Squire 10 Fleet Street**
**London EC4Y 1AY (GB)**

(54) **Human Fc-gamma receptor.**

(57) A cDNA clone is provided which encodes a human receptor for the Fc portion of immunoglobulin G. Soluble forms of the receptor may be useful in treating disorders associated with excessive immunoglobulin G production. Cells expressing membrane-bound forms of the receptor are useful in assays for immune complexes, elevated levels of which are associated with numerous disease states, including systemic lupus erythematosus (SLE) and rheumatoid arthritis.

EP 0 319 307 A2

## Description

## HUMAN Fc-gamma RECEPTOR

### Field of The Invention

The invention relates generally to methods for treating and diagnosing immune disorders, and more particularly to the use of expression products of a human immunoglobulin G (IgG) receptor cDNA to treat and diagnose conditions associated with elevated levels of immunoglobulin G, either in monomeric or in aggregated form.

### BACKGROUND

Both exogenous and endogenous antigens can trigger pathogenic immune responses which result in elevated serum levels of aggregates consisting of immunoglobulin and antigen. These aggregates are referred to as immune complexes. Elevated levels of immune complexes are correlated with a wide variety of autoimmune diseases, such as systemic lupus erythematosus (SLE) and rheumatoid arthritis, certain neoplasias, and complications of infectious diseases due to bacteria, viruses, and parasites. As a result, the measurement of the serum level of immune complexes is an important diagnostic tool, particularly for certain autoimmune disorders: e.g. Theofilopoulos et al., Am. J. Pathol., Vol. 100, pgs. 531-591 (1980).

Present assays for the serum level of immune complexes include solid-phase assays which take advantage of the affinity of the complexes for certain complement or rheumatoid factor proteins, and cellular assays which take advantage of the property of Raji cells to bind immune complexes preferentially: Theofilopoulos et al., chapter 28, and Toth et al., chapter 29, in Rose et al., eds. Manual of Clinical Immunology, 3rd Ed. (American Society for Microbiology, Washington, D.C., 1986).

Receptors for the Fc region of IgG (FcγR) are expressed in a number of hematopoietic cell types and play important roles in several immunological processes such as phagocytosis of opsonized particulate antigens (Mellman et al., J. Cell. Biol., Vol. 96, pgs. 887-895 (1983)), clearance of immune complexes (Kurlander et al., J. Immunol., Vol. 133, pgs. 855-862 (1984)), and also antibody-dependent cellular cytotoxicity, signalling the production of inflammatory mediators, and regulation of immunoglobulin synthesis.

Receptors for human IgG have been partially characterized as at least three distinct molecular species which are expressed on different but overlapping subsets of hematopoietic cells. These FcγR classes have been termed FcγRI, FcγRII and FcγR$_{lo}$ ("lo" indicates "low affinity"), and have been distinguished on the basis of size, affinity for subclasses of human and mouse IgG, distribution of expression on various cell types, in vitro functionality such as mediation of anti-T3 T-lymphocyte proliferation, and recognition by monoclonal antibodies; see the review by Anderson et al., Immunol. Today, Vol. 7, pgs. 264-266 (1986). FcγRI is a 70 kd receptor expressed on human monocytes which exhibits a relatively high affinity for monomeric IgG ($10^8$-$10^9$ M$^{-1}$). FcγRII is a 40 kd species present on monocytes, B cells, neutrophils, platelets, and eosinophils which has a much lower affinity for ligand than Fcγl, preferring aggregated IgG (Jones et al., J. Immunol., Vol. 135, pgs. 3348-3351 (1985), and J. Immunol., Vol. 136, pgs. 1641-1647 (1986). FcγR$_{lo}$, from neutrophils, eosinophils, T cells, and macrophages, is detected as a broad band of 50-70 kd, and apparently also binds only aggregated ligand.

It has been shown that a soluble form (i.e. non-membrane-bound form) of FcγR suppresses the production of serum IgG in humans: Bich-Thuy et al., J. Immunol., Vol. 129, pgs. 150-152 (1982); Samarut et al., Eur. J. Immunol., Vol. 10, pgs. 352-358 (1980); Bich-Thuy et al., Eur. J. Immunol., Vol. 15, pgs. 96-99 (1985); and Samarut et al., Eur. J. Immunol., Vol. 10, pgs. 894-898 (1980). We believe that this property could be exploited to ameliorate the adverse effects of several disease conditions, such as some myelomas, that are characterized by excessive IgG production. Administration of soluble FcγR may down-regulate IgG production, thereby ameliorating adverse effects, such as kidney damage, that are associated with high levels of immunoglobulin.

In light of the foregoing, it would be advantageous for the medical community to have alternative assay methods for immune complexes utilizing well-characterized, widely available, and conveniently cultured cell lines. It would also be advantageous if soluble FcγRs could be produced in sufficient quantity to permit therapeutic applications.

### SUMMARY OF THE INVENTION

The present invention is directed to nucleic acids capable of encoding a receptor for the Fc portion of immunoglobulin G (IgG), both soluble and membrane-bound forms. The invention also includes polypeptides corresponding to the soluble and membrane-bound forms of the receptor, and methods of using those polypeptides to suppress serum IgG levels, and to measure serum immune complex levels. The polypeptides of the invention are referred to as Fc-gamma receptors or FcγRS.

The invention is based on the discovery of cDNA encoding human FcγR. A cDNA clone containing the FcγR of the invention, pcD-hFcγR-16.2, is deposited with the American Type Culture Collection, Rockville, MD, USA, under accession number 67565.

### Brief Description of the Drawings

Figure 1 diagrammatically illustrates the pcD plasmid carrying the cDNA insert for the FcγR of the invention; and

Figure 2 illustrates the nucleotide sequence of the cDNA insert of plasmid pcD-hFcγR-16.2.

## DETAILED DESCRIPTION OF THE INVENTION

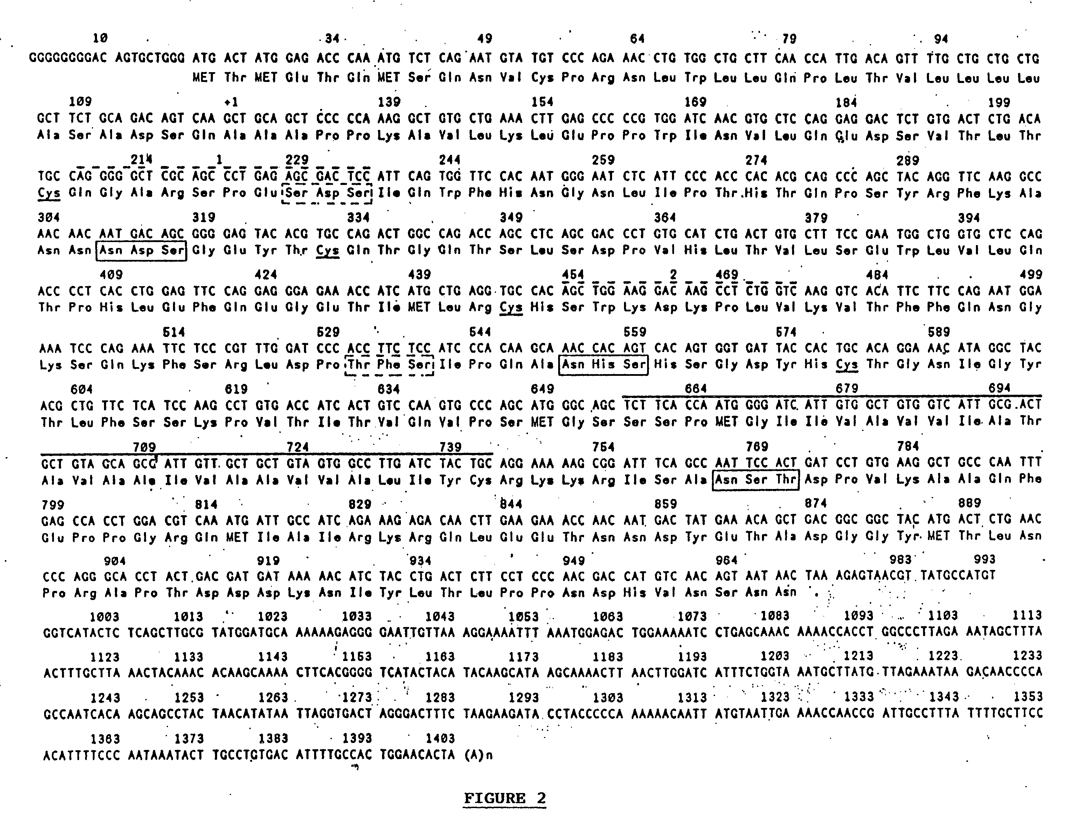

The present invention includes nucleic acids encoding polypeptides capable of binding to the Fc portion of human IgG. The nucleic acids are defined by the open reading frame of the cDNA insert illustrated in Figure 2. The invention also includes soluble and membrane-bound polypeptides for the human FcγR. These and other modified versions of the polypeptides are readily produced using standard protein-engineering techniques.

Once nucleic acid sequence and/or amino acid sequence information is available for a native protein, a variety of techniques become available for producing virtually any mutation in the native sequence. Shortle, in Science, Vol. 229, pgs. 1193-1201 (1985), reviews techniques for mutating nucleic acids which are applicable to the present invention. Preferably, mutants of the protein of the present invention are produced by site-specific oligonucleotide-directed mutagenesis: e.g. Zoller and Smith, Methods in Enzymology, Vol. 100, pgs. 468-500 (1983); Mark et al., U.S. Patent 4,518,584 entitled "Human Recombinant Interleukin-2 Muteins", which are incorporated by reference; or by so-called "cassette" mutagenesis described by Wells et al., in Gene, Vol. 34, pgs. 315-323 (1985), and by Estell et al., Science, Vol. 233, pgs. 659-663 (1986), and also essentially by Mullenbach et al., J. Biol. Chem., Vol. 261, pgs. 719-722 (1986), and by Feretti et al., Proc. Natl. Acad. Sci., Vol. 83, pgs. 597-603 (1986).

Polypeptides with amino acid modifications (i.e. muteins) may be desirable in a variety of circumstances. For example, undesirable side effects might be reduced by certain muteins, particularly if the side-effect activity is associated with a different part of the polypeptide from that of the desired activity. In some expression systems, the native polypeptide may be susceptible to degradation by proteases. In such cases, selected substitutions and/or deletions of amino acids which change the susceptible sequences can significantly enhance yields: e.g. British patent application 2,173,804-A where Arg at position 275 of human tissue plasminogen activator is replaced by Gly or Glu. Muteins may also increase yields in purification procedures and/or increase shelf lives of proteins by eliminating amino acids susceptible to oxidation, acylation, alkylation, or other chemical modifications. For example, methionines readily undergo oxidation to form sulfoxides, which in many proteins is associated with loss of biological activity: e.g. Brot and Weissbach, Arch. Biochem. Biophys., Vol. 223, pg. 271 (1983). Often methionines can be replaced by more inert amino acids with little or no loss of biological activity: e.g. Australian patent application AU-A-52451/86. In bacterial expression systems, yields can sometimes be increased by eliminating or replacing conformationally inessential cysteine residues: e.g. Mark et al., U.S. Patent 4,518,584.

Preferably, soluble forms of the FcγR of the invention are produced by introducing a stop codon prior to (i.e. in the 5'- or "upstream" direction of) the coding region for the transmembrane and intracellular portions of the FcγR cDNA. This is conveniently done by site-specific mutagenesis.

The nucleotide sequence of the cDNA insert of clone pcD-hFcγR-16.2 and the deduced amino acid sequence are illustrated in Figure 2. The NH₂-terminal signal sequence is about 30 amino acids in length and is followed by an extracellular domain of about 180 amino acids. "+1" above the nucleotide sequence indicates the start of the extracellular domain, determined by analogy with the mouse sequence for FcγR. Three potential N-linked glycosylation sites are boxed. Two glycosylation sites which have not been conserved between the human and mouse sequences are indicated by broken boxes. Cysteine residues which have potential to form intrachain disulfide bonds are underlined. The putative transmembrane domain has been indicated with an overbar beginning at nucleotide 656. The carboxy-terminal cytoplasmic domain consists of 76 amino acids, terminates at nucleotide residue 973 and contains one potential site for N-linked glycosylation.

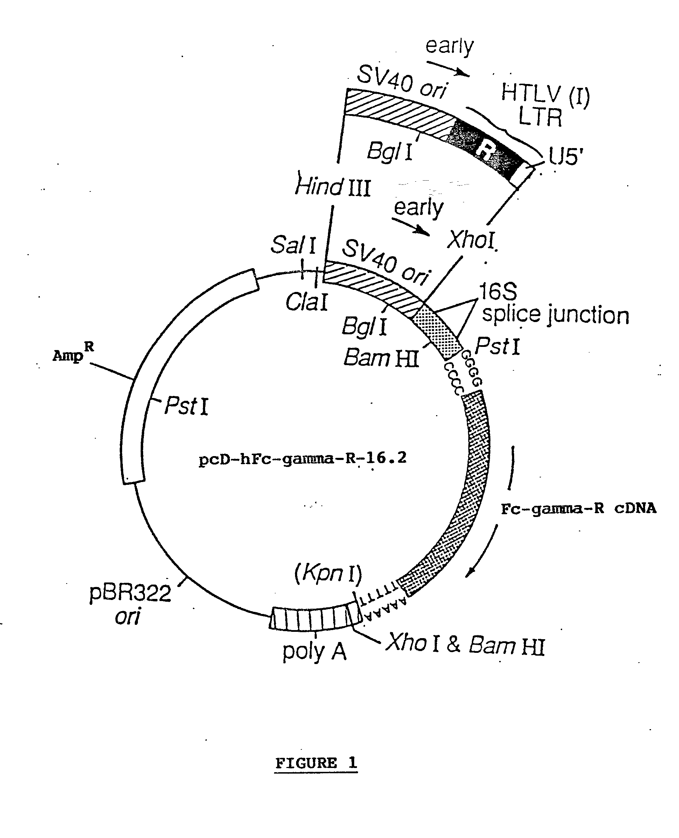

Plasmid pcD-hFcγR-16.2 is illustrated diagrammatically in Figure 1. The vector is similar to the pcD shuttle vector described by Okayama and Berg, Mol. Cell. Biol., Vol. 2, pgs. 161-170 (1983), and Vol. 3, pgs. 280-289 (1983), except that the SV40 promoter has been modified to improve expression by the downstream insertion of a portion of the long terminal repeat (LTR) from an HTLV(I) retrovirus. The plasmid is conveniently propagated in E. coli K12 strain MC1061, or like host.

The immunoglobulin G binding property of the FcγRs of the invention is measured by standard techniques, e.g. (1) the ability of cells transfected with the FcγR cDNA to form rosettes in the presence of IgG-coated red blood cells (RBCs), to preferentially bind human IgG aggregated by heat treatment, in the case of membrane-bound FcγR; or (2) the ability to preferentially remove FcγR from solution by an immunoadsorbent column comprising human IgG, or to inhibit rosette formation between IgG-coated RBCs and cells known to have FcγRs, in the case of soluble FcγR. The former measurements can be made with fluorescently labeled human IgG molecules: e.g. Haugland, Handbook of Fluorescent Probes (Molecular Probes, Inc., Junction City, OR, 1985). The latter measurements can be made by constructing an IgG column from isolated human IgG and a commercially available activated sepharose column, e.g. from Bio-Rad Laboratories (Richmond, CA).

Rosette assays are standard in the art: e.g. Winchester et al., chapter 31, in Roce et al., eds., Manual of Clinical Laboratory Immunology, 3rd Ed. (American Society for Microbiology, Washington, D.C., 1986).

Once the cDNA of the invention has been cloned, a wide range of expression systems (i.e. host-expression vector combinations) can be used to produce the proteins of the invention. Possible types of host cells include, but are not limited to, bacterial, yeast, insect, mammalian, and the like. Selecting an expression system, and optimizing protein production thereby, involves the consideration and balancing of many factors, including (1) the nature of the protein to be expressed: e.g. the protein may be poisonous to some host organisms, it may be susceptible to degradation by host proteases, or it may be expressed in inactive conformations or in insoluble form in some hosts; (2) the nature of the messenger RNA (mRNA) corresponding to the protein of interest: e.g. the mRNA may have sequences particularly susceptible to host endonucleases, which drastically reduce the functional lifetime of the mRNA, or the mRNA may form secondary structures that mask the start codon or ribosome binding site, thereby inhibiting initiation of translation in some hosts; (3) the selection, availability, and arrangement of host-compatible expression control sequences in the 3'- and 5'-regions flanking the coding region -- these include promoters, 5'- and 3'-protector sequences, ribosome binding sites, transcription terminators, enhancers, polyadenylate addition sites, cap sites, intron-splice sites, and the like; (4) whether the protein has a secretion signal sequence which can be processed by the host, or whether an expression control sequence encoding a signal sequence endogenous to the host must be spliced onto the region encoding the mature protein; (5) the available modes and efficiencies of transfection or transformation of the host, and whether transient or stable expression is desired; (6) the scale and cost of the host culture system desired for expressing the protein; (7) whether, and what type of, posttranslational modifications are desired, e.g. the extent and kind of glycosylation desired may affect the choice of host; (8) the ease with which the expressed protein can be separated from proteins and other materials of the host cells and/or culture medium: e.g. with some hosts or expressed proteins it may be desirable to express a fusion protein with a specialized signal sequence to aid in later purification steps (e.g. Sassenfeld et al., Biotechnology, January 1984); (9) the stability and copy number of a particular vector in a selected host: e.g. Hofschneider et al., eds. Gene Cloning in Organisms Other than E. Coli (Springer Verlag, Berlin, 1982); and (10) like factors known to those skilled in the art.

Many reviews are available which provide guidance for making choices and/or modifications of specific expression systems in light of the recited factors: e.g. de Boer and Shepard, in "Strategies for Optimizing Foreign Gene Expression in Escherichia coli," pgs. 205-247, in Kroon, ed. Genes: Structure and Expression (John Wiley & Sons, New York, 1983), review several E. coli expression systems; Kucherlapati et al., in Critical Reviews in Biochemistry, Vol. 16, Issue 4, pgs. 349-379 (1984), and Banerji et al., in Genetic Engineering, Vol. 5, pgs. 19-31 (1983) review methods for transfecting and transforming mammalian cells; Reznikoff and Gold, eds., in Maximizing Gene Expression (Butterworths, Boston, 1986) review selected topics in gene expression in E. coli, yeast, and mammalian cells; and Thilly, in Mammalian Cell Technology (Butterworths, Boston, 1986) reviews mammalian expression systems.

Likewise, many reviews are available which describe techniques and conditions for linking and/or manipulating specific cDNAs and expression control sequences to create and/or modify expression vectors suitable for use with the present invention: e.g. Maniatis et al., Molecular Cloning: A Laboratory Manual (Cold Spring Harbor Laboratory, N.Y., 1982); Glover, DNA Cloning: A Practical Approach, Vol. I and II (IRL Press, Oxford, 1985), and Perbal, A Practical Guide to Molecular Cloning (John Wiley & Sons, N.Y., 1984), to name only a few. Generally, within an expression vector various sites may be selected for insertion of the cDNA of the invention. These sites are usually designated by the restriction endonuclease which cuts there and are well recognized by those of skill in the art. Various methods for inserting DNA sequences into these sites to form recombinant DNA molecules are also well known. These include, for example, dG-dC or dA-dT tailing, direct ligation, synthetic linkers, exonuclease and polymerase-linked repair reactions followed by ligation, or extension of the DNA strand with DNA polymerase and an appropriate single-stranded template followed by ligation.

Often a vector containing the cDNA of the invention must be obtained in large quantities before transfection and/or transformation of cells in a host culture can take place. For this purpose the vector is often replicated without significant expression in an organism (the cloning host) other than the one finally used for expression. In such cases, after propagation, the vectors are separated from the cloning host using standard techniques, e.g. as disclosed by Maniatis et al. (cited above).

In connection with the techniques for manipulating, constructing, and cloning DNAs the following terms are employed:

"Digestion" of DNA refers to catalytic cleavage of the DNA usually with an enzyme that acts only at certain locations in the DNA. For the most part such enzymes are restriction endonucleases, and the sites on the DNA for which each is specific is called a restriction site. The various restriction enzymes used herein are commercially available, and their reaction conditions, cofactors, and other requirements as established by the enzyme suppliers are used. Generally, about 1 microgram of plasmid or DNA fragment is used with about 1 unit of enzyme in about 20 microliters of buffer solution. Appropriate buffer and substrate amounts for particular restriction enzymes are specified by the manufacturer. Incubation times of about 1 hour at 37°C are ordinarily used, but may vary in accordance with the supplier's instructions. After incubation, protein is removed by extraction with phenol and chloroform, and the digested nucleic acid is recovered from the aqueous fraction by precipitation with ethanol.

"Isolation" or "purification" of a given DNA fragment from a restriction (or other) digest means separation of the digest by polyacrylamide gel or agarose gel electrophoresis, identification of the fragment of interest by comparison of its mobility versus that of marker DNA fragments of known molecular weight, removal of the gel section containing the desired fragment, and separation of DNA from the gel. Isolation procedures are well known: e.g. Lawn et al., Nucleic Acids Research, Vol. 9 pgs. 6103-6114 (1981); Goeddel et al. Nucleic Acids Research, Vol. 8, pg. 4057 (1980); and Maniatis et al. (cited above).

"Ligation" refers to the process of forming phosphodiester bonds between the double-stranded nucleic acid fragments. Ligation may be accomplished using known buffers and conditions with 10 units of T4 DNA ligase per 0.5 micrograms of approximately equimolar amounts of the DNA fragments to be ligated.

"Amplification" of a plasmid means transforming a suitable cloning host and propagating the host to increase the total number of plasmids.

"Kinased" DNA fragments refer to fragments which have been phosphorylated with polynucleotide kinase. Such treatment enhances the efficiency of ligation of DNA fragments lacking 5'-phosphates.

Suitable prokaryote expression vectors include plasmids from E. coli, e.g. Col. E1, pCR1, pBR322, pMB9 and their derivatives, plasmids suitable for a wider range of hosts, e.g. RP4, phage DNAs, such as phage lambda and its various derivatives, M13, and the like. Additional E. coli vectors are described in Chapter 12 of Maniatis et al. (cited above) for expressing eukaryotic proteins, either fused or unfused with prokaryotic peptides. Riggs discloses still further E. coli expression systems in U.S. Patent 4,431,739, which is incorporated by reference.

Commonly used prokaryotic promoters include the β-lactamase (penicillinase) and lactose promoter systems (Chang et al., Nature, Vol. 275, pg. 615 (1978); Itakura, et al., Science, Vol. 198, pg. 1056 (1977); Goeddel et al., Nature Vol. 281, pg. 544 (1979)); and the tryptophan (trp) promoter system (Goeddel, et al., Nucleic Acids Res., Vol. 8, pg. 4057 (1980); EPO Appl Publ No. 0036776). Although these are the most commonly used, other microbial promoters have been discovered and utilized, and details concerning their nucleotide sequences have been published, enabling a skilled worker to ligate them functionally with plasmid vectors: e.g. Siebenlist et al., Cell, Vol. 20, pg. 269 (1980).

A particularly useful prokaryotic promoter for high expression in E. coli is the tac promoter, disclosed by de Boer in U.S. Patent 4,551,433, which is incorporated here in by reference. Secretion expression vectors are also available for E. coli hosts. Particularly useful are the pIN-III-ompA vectors, disclosed by Ghrayeb et al. in EMBO J., Vol. 3, pgs. 2437-2442 (1984), in which the cDNA to be transcribed is fused to the portion of the E. coli ompA gene encoding the signal peptide of the ompA protein which, in turn, causes the mature protein to be secreted into the periplasmic space of the bacteria. Likewise, U.S. Patents 4,336,336 and 4,338,397 disclose secretion expression vectors for prokaryotes. Accordingly, these references are incorporated by reference.

Numerous strains of bacteria are suitable hosts for prokaryotic expression vectors including strains of E. coli, such as W3110 (ATCC No. 27325), JA221, C600, ED767, DH1, LE392, HB101, X1776 (ATCC No. 31244), X2282, RR1 (ATCC No. 31343) MRCI; strains of Bacillus subtilis; and other enterobacteriaceae such as Salmonella typhimurium or Serratia marcescens, and various species of Pseudomonas. General methods for deriving bacterial strains, such as E. coli K12 X1776, useful in the expression of eukaryotic proteins is disclosed by Curtis III in U.S. Patent 4,190,495. Accordingly this patent is incorporated by reference.

Eukaryotic microbes, such as yeast cultures, can also be used to express proteins of the invention. Saccharomyces cerevisiae, or common baker's yeast, is the most commonly used among eukaryotic microorganisms, although a number of other strains are commonly available. For expression in Saccharomyces, the plasmid YRp7 can be used: e.g. Stinchcomb et al, Nature, Vol. 282, pg. 39 (1979); Kingsman et al, Gene, Vol. 7, pg. 141 (1979); and Tschemper et al., Gene, Vol. 10, pg. 157 (1980). This plasmid already contains the trp1 gene which provides a selection marker for a mutant strain of yeast lacking the ability to grow in tryptophan, for example ATCC No. 44076 or PEP4-1 (Jones, Genetics, Vol. 85, pg. 12 (1977)). The presence of the trp1 lesion as a characteristic of the yeast host cell genome then provides an effective environment for detecting transformation by growth in the absence of tryptophan. Additional vectors for yeast include the pGAL plasmids disclosed by Miyajima et al., Nucleic Acids Research, Vol. 12, pgs. 6397-6414 (1984), and the 2μm plasmid disclosed by Beggs, Nature, Vol. 275, pgs. 104-109 (1978).

Suitable promoting sequences in yeast vectors include the promoters for 3-phosphoglycerate kinase (Hitzeman et al., J. Biol. Chem., Vol. 255, pg. 2073 (1980)) or other glycolytic enzymes (Hess et al, J. Adv. Enzyme Reg., Vol. 7 pg. 149 (1968); Holland et al, Biochemistry, Vol. 17, pg. 4900 (1978)), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triose-phosphate isomerase, phosphoglucose isomerase, and glucokinase. In constructing suitable expression plasmids, the termination sequences associated with these genes are also ligated into the expression vector adjacent to the 3'- end of the sequence to be expressed to provide polyadenylation and termination. Other promoters, which have the additional advantage of transcription controlled by growth conditions, are the promoter regions for alcohol dehydrogenase 2, isocytochrome C, acid phosphatase, degradative enzymes associated with nitrogen metabolism, and the aforementioned glyceraldehyde-3-phosphate dehydrogenase, and enzymes responsible for maltose and galactose utilization (Holland, cited above). Yet another regulatable promoter is the metallothionein promoter system disclosed by Fogel et al. in U.S. Patent 4,511,652, incorporated herein by reference. Virtually any plasmid vector containing a promoter, origin of replication and termination sequences that are all yeast-compatible, is suitable.

Secretion expression vectors are also available for S. cerevisiae hosts: e.g. pMF α8 disclosed by Miyajima et al., Gene Vol. 37, pgs. 155-161 (1985); YEpIPT disclosed by Hitzeman et al., Science, Vol. 219, pgs. 620-625 (1983); pYαEGF-21 disclosed by Brake et al., Proc. Natl. Acad. Sci., Vol. 81, pgs. 4642-4646 (1984) and by Brake in European Patent Application 0116201; and plasmids disclosed by Singh in European Patent Application 0123544.

In addition to prokaryotic and eukaryotic microorganisms, expression systems comprising cells derived from multicellular organisms may also be used to produce proteins of the invention. Of particular interest are mammalian expression systems because their post-translational processing machinery is more likely to produce biologically active mammalian proteins. Several DNA tumor viruses have been used as vectors for mammalian hosts; e.g. see Tooze, ed., DNA Tumor Viruses, 2nd Ed. (Cold Spring Harbor Laboratory, N.Y., 1981) for a review of their biology. Particularly important are the numerous vectors which comprise SV40 replication sequences, transcription sequences, and/or translation control sequences coupled to bacterial replication control sequences: e.g. the pcD vectors developed by Okayama and Berg and disclosed in Mol. Cell. Biol., Vol. 2, pgs. 161-170 (1982) and in Mol. Cell. Biol., Vol. 3, pgs. 280-289 (1983), both of which are incorporated herein by reference; the SV40 vectors disclosed by Hamer in Genetic Engineering, Vol. 2, pgs. 83-100 (1980), and U.S. Patent 4,599,308, both of which are incorporated herein by reference; and the vectors additionally containing adenovirus regulatory elements, disclosed by Kaufman and Sharp, in Mol. Cell. Biol., Vol. 2, pgs. 1304-1319 (1982), and by Clark et al., U.S. Patent 4,675,285, both of which are incorporated herein by reference. Monkey cells are usually the preferred hosts for the above vectors. Such vectors containing the SV40 ori sequences and an intact A gene can replicate autonomously in monkey cells (to give higher copy numbers and/or more stable copy numbers than nonautonomously replicating plasmids). Moreover, vectors containing the SV40 ori sequences without an intact A gene can replicate autonomously to high copy numbers (but not stably) in COS7 monkey cells, described by Gluzman, Cell, Vol. 23, pgs. 175-182 (1981) and available from the ATCC (accession no. CRL 1651). The above SV40-based vectors are also capable of transforming other mammalian cells, such as mouse L cells, by integration into the host cell DNA.

Besides the SV40-based vectors, mammalian expression systems suitable for use with the present invention include, but are not limited to, (i) vectors comprising bovine papilloma virus (BPV) sequences; e.g. BPV-pBR322 hybrids disclosed by Sarver et al., Genetic Engineering, Vol. 5, pgs. 173-190 (1983); and by DiMaio et al., Proc. Natl. Acad. Sci., Vol. 79, pgs. 4030-4034 (1982) for transforming bovine and mouse cells; (ii) vectors comprising Epstein-Barr virus (EBV) sequences: e.g. plasmids carrying the EBV oriP sequences (including the coding sequences for the nuclear antigen EBNA-1) disclosed by Yates et al., Nature, Vol. 313, pgs. 812-815 (1985), by Reisman et al., Mol. Cell. Biol., Vol. 5, pgs. 1822-1832 (1985), by Yates et al., Proc. Natl. Acad. Sci., Vol. 81, pgs. 3806-3810 (1984), and by Sugden et al., Mol. Cell. Biol., Vol. 5, pgs. 410-413 (1985) for stable transformation of various mammalian cells including human and monkey cells; (iii) vectors comprising murine polyoma virus sequences, e.g. O'Hara, J. Mol. Biol., Vol. 151, pg. 203 (1981) for transforming mouse and hamster cells; (iv) vectors carrying the dihydrofolate reductase (dhfr) gene, e.g. Alt et al., J. Biol. Chem., Vol. 253, pgs. 1357-1370 (1978), which in response to methotrexate treatment duplicates together with adjacent coding regions cointegrated into murine genomes (e.g., that of a Chinese hamster ovary (CHO) cell line deficient in dhfr activity) e.g. as described by Urlamb et al., Proc. Natl. Acad. Sci., Vol. 77, pg. 4216 (1980); and (v) cotransformation systems such as that disclosed by Axel et al. in U.S. Patent 4,399,216. Additional mammalian expression vectors can be constructed, or existing ones modified, by using various elements from available DNA tumor viruses and retroviruses, e.g. origins of replication, enhancer sequences (such as the long terminal repeat sequence from Rous sarcoma virus (RSV-LTR) disclosed by Gorman et al., Proc. Natl. Acad. Sci., Vol. 79, pgs. 6777-6781 (1982)), intron-splice sites, polyadenylation sites, and the like.

Invertebrate expression systems can also be constructed for use with the invention, e.g. the larvae of silk worm, Bombyx mori, infected by a baculovirus vector, BmNPV, described by Maeda et al. in Nature, Vol. 315, pgs. 892-894 (1985), and in Saibo Koguku, Vol. 4, pgs. 767-779 (1985).

The polypeptides of the present invention expressed in E. coli, in yeast or in other cells can be purified according to standard procedures of the art, including precipitation with ammonium sulfate, fractionation column chromatography (e.g., ion exchange, gel filtration, electrophoresis, affinity chromatography, etc.) and ultimately crystallization (see generally "Enzyme Purification and Related Techniques," Methods in Enzymology, 22:233-577 [1977] and Scopes, R., Protein Purification: Principles and Practice, Springer-Verlag, New York [1982]). Once purified, partially or to homogeneity, the polypeptides of the invention may be used for research purposes, e.g., as an antigenic substance for eliciting specific immunoglobulins useful in immunoassays, immunofluorescent stainings, etc. (see generally, Immunological Methods, Vols. I & II Eds. Lefkovits, I. and Pernis, B., Academic Press, New York, N.Y. [1979 & 1981], and Handbook of Experimental Immunology, ed. Weir, D., Blackwell Scientific Publications, St. Louis, MO [1978]).

The polypeptides of the present invention may also be used in pharmaceutical compositions, e.g., to suppress IgG production. Thus, patients with certain autoimmune diseases, myeloma, or the like, may be treated directly with such polypeptides.

Pharmaceutical compositions containing the polypeptides of this invention are prepared by admixing or compounding these polypeptides with preferably inert, pharmaceutically acceptable carriers. Suitable carriers and processes for their preparation are well known in the art (see, e.g., Remington's Pharmaceutical Sciences and U.S. Pharmacopeia: National Formulary, Mack Publishing Company, Easton, PA [1984]). The preferred course of administration is parenteral and can include use of mechanical delivery systems.

Preferably, the pharmaceutical composition is in unit dosage form. In such form, the preparation is subdivided into unit doses containing appropriate quantities of the active component. The quantity of active compound in a unit dose of preparation may be varied or adjusted from 1 μg to 100 mg, according to the particular application and the potency of the active ingredient. The composition can, if desired, also contain other therapeutic agents.

The dosage may be varied depending upon the requirement of the patient, the severity of the condition being treated and the particular compound being employed. The term "effective amount" as used herein is meant to take these factors into account when dosages are considered. Determination of the proper dosage for a particular situation is within the skill of the art. Generally, treatment is initiated with smaller dosages which are less than the optimum dose of the compound. Thereafter, the dosage is increased by small amounts until the optimum effect under the circumstances is reached. For convenience, the total daily dosage may be divided and administered in portions during the day.

## EXAMPLES

The following examples serve to illustrate the present invention. Selection of vectors and hosts as well as the concentration of reagents, temperatures, and the values of other variables are only to exemplify application of the present invention and are not to be considered limitations thereof.

Example I. Construction of stable mammalian cell transformants which express human FcγR.

Preferably, mammalian cell lines capable of stable expression of the FcγR are produced by cotransfecting a host mammalian cell with a vector carrying a selectable marker and a vector carrying a host-compatible promoter and the FcγR cDNA insert. For pcD-hFcγR-16.2, hosts include Chinese hamster ovary cells and mouse L cells, such as a thymidine kinase deficient mutant (td⁻) L cell available from the American Type Culture Collection under accession number CCL 1.3. The selectable marker allows one to select host cells which have a high probability of containing the FcγR gene fully integrated into the host genome. Typically, the ratio of pcD-hFcγR-16.2 to the marker-containing vector in the transfection solution is about 10:1. Thus, if the marker gene is integrated into the host genome, it is very likely that pcD-hFcγR-16.2 will also be integrated by virtue of its higher concentration. The selectable marker also provides a means of preventing the cultures of desired transformants from being overgrown by revertant cells.

tk⁻ mouse L cells were cotransfected with pcD-hFcγR-16.2 and pSV2tk, a pSV2 plasmid carrying a thymidine kinase gene under control of the SV40 early promoter. The pSV2 plasmid is described by Mulligan et al., Science, Vol. 209, pgs. 1422-1427 (1980), and by Subramani et al., Mol. Cell. Ciol., Vol. 1, pgs. 854-864 (1981), and is available from the American Type Culture Collection under accession number 37146. Both plasmids are amplified in E. coli, e.g. strain HB101 available from the ATCC under accession number 33694, and purified by cesium chloride equilibrium centrifugation. A suspension of about $1 \times 10^5$ of tk⁻ L cells in 10 ml of Dulbecco's Modified Eagle medium (DME) with 10% fetal bovine serum is placed in a Falcon 3003 dish and cultured at 37°C for 20 hours in a 5% carbon dioxide gas incubator, after which the medium is replaced by 10 ml of fresh DME with 10% fetal bovine serum. The culture is incubated for an additional 4 hours. After incubation 0.5 ml of soluble A (50 mM Hepes, 280 mM NaCl, 1.5 mM sodium phosphate buffer, pH 7.22) and 0.5 ml of solution B (2M $CaCl_2$, 10 μg pcD-FcγR-16.2, 1 μg pSV2tk) are added to the culture medium, and the culture is incubated at 37°C for 24 hours in a 5% $CO_2$ atmosphere, after which the cells are placed in a selective medium with HAT (e.g. Sigma Chemical Co., St. Louis, MO). After two weeks the surviving colonies are subcloned by limiting dilution, and clones are assayed for expression of FcγR.

Example II. Use of stable L cell transformant expressing membrane-bound FcγR to measure serum levels of immune complex

A stably transformed mammalian cell expressing the FcγR of the invention can replace the Raji cell line in assays for immune complexes, e.g. Theofilopoulos et al., chapter 28, Manual of Clinical Laboratory Immunology, 3rd Ed. (American Society for Microbiology, Washington, D.C. 1986).

Antiserum to human IgG is prepared in rabbits, and the IgG fraction is isolated by precipitation with ammonium sulfate, followed by fractionation on an anion-exchange chromatography column (DEAE-cellulose 52; Whatman Chemical Separation Ltd., Maidstone, England). Antiserum from commercial sources may also be used. The IgG fraction of the antiserum is brought to 5 mg/ml, and 1 ml is labeled with ¹²⁵I. After iodination and dialysis, the antiserum is diluted to 1 mg/ml with phosphate-buffered saline (PBS) to give a specific activity of about $3 \times 10^5$ cpm/μg. Cells transformed with the FcγR cDNA are harvested after 72 h of culture, and portions of $2 \times 10^6$ cells in 200 μl of medium are placed in 1.5-ml plastic Eppendorf conical tubes (Brinkmann Instruments, Inc., Westbury, N.Y.; catalog no. 22-36-411-1). One milliliter of Spinner medium (Eagle minimal medium without $Ca^{2+}$ and $Mg^{2+}$) is added to each tube, and the cells are centrifuged at 800 x g for 8 min. Supernatant fluids are aspirated, and the cell pellets are resuspended in 50 μl of Spinner medium. Serum to be tested is diluted fourfold in 0.15 M NaCl (physiological saline), and 25 μl is added to the transformed cells. After a 45-min incubation period at 37°C with gentle shaking by hand every 5 to 10 min, the cells are washed three times with Spinner medium. After the final wash, the cells are allowed to react for 30 min at 4°C, with gentle shaking every 5 to 10 min, with $^{125}I$-labeled rabbit anti-human IgG diluted with Spinner medium containing 10 g of human serum albumin (HSA) per liter. After incubation, the cells are washed three times, supernatant fluids are completely aspirated, and cell pellet radioactivity is determined in a gamma counter. All assays are done in duplicate. The amount of uptake expressed as absolute counts, percentage of the input, or micrograms of antibody, is referred to a standard curve of radioactive antibody uptake by cells incubated with normal human serum (complement source) containing various amounts of aggregated human IgG (AHG). The quantity of immune complex in serum is equated to an amount of AHG after correction for the dilution factor and is expressed as micrograms of AHG equivalent per milliliter of serum. The soluble AHG is formed from a solution of 6.5 mg of Cohn fraction II or isolated human IgG per ml of physiological saline, heated at 63°C for 30 min, and centrifuged (1,500 x g, 15 min) to remove insoluble large aggregates. This preparation is then diluted with buffer to obtain a final concentration of approximately 1.6 mg/ml. Portions (0.5 ml) of this preparation are stored at -70°C and can be used up to 1 month later.

The standard curve of radioactive antibody uptake is constructed as follows. Fifty-microliter portions of AHG (80 µg of protein) are serially diluted (11 twofold dilutions) in saline. Subsequently, 50 µl of a two-fold dilution of normal human serum (source of complement), freshly obtained or stored at -70°C, is added to each dilution of AHG, mixed carefully, and incubated at 37°C for 30 min. Thereafter, 25 µl of each mixture is added to 2 x 10$^6$ cells in duplicate (fourfold final dilutions of serum containing from 20 µg to about 20 ng of AHG); the mixture is incubated, washed, and reacted with radiolabeled antibody. Radioactivity is then counted as with the test sera. A base line of radioactive antibody uptake (background) by cells incubated with 25 µl of a fourfold dilution of normal human serum, used as a source of complement in the reference curve, is also established.

Standard preparations of IgG aggregates and of tetanus toxoid-human anti-tetanus toxoid immune complexes have recently been developed under the auspices of the International Union of Immunologists and are available through the Swiss Red Cross Blood Transfusion Service, e.g. Nydegger et al., Clin. Exp. Immunol., Vol. 58, pgs. 502-509 (1984).

Example III. Construction of soluble form of human FcγR.

A soluble FcγR was constructed using site-specific oligonucleotide-directed mutagenesis of the FcγR cDNA insert of pcD-hFcγR-16.2. The entire cDNA insert of pcD-hFcγR-16.2 was subcloned as a 1.7 kilobase Xhol fragment into the Bluescript KS plasmid (Stratagene, San Diego, CA) and single-stranded DNA was then prepared. A synthetic oligonucleotide encoding a TGA stop codon and a Xhol site was used as a primer for complementary-strand synthesis using DNA polymerase I large fragment Amersham, Arlington Heights, IL). This produced a mutant lacking both the cytoplasmic and transmembrane domains by converting the Ser at nucleotide positions 656-658 into a stop codon. After the identity of the mutant was confirmed by DNA sequence analysis, it was subcloned back into the Xhol site of the pcD vector, amplified in E. coli, and transfected into COS 7 cells using the following protocol. One day prior to transfection, approximately 10$^6$ COS 7 monkey cells were seeded onto individual 100 mm plates in Dulbecco's modified Eagle's medium (DME) containing 10% fetal calf serum and 2 mM glutamine. To perform the transfection, the medium was aspirated from each plate and replaced with 4 ml of DME containing 50 mM Tris.Hcl pH 7.4, 400 µg/ml DEAE-Dextran, 100 µM chloroquine, and 10 µg of the plasmid DNAs to be tested. The plates were incubated for four hours at 37°C, then the DNA-containing medium was removed, and the plates were washed twice with 5 ml of serum-free DME, then with DME containing 4% fetal calf serum, 2 mM glutamine, and penicillin, and streptomycin was added. The cells were then incubated for 72 hrs at 37°C. The soluble receptor in the culture medium exhibited affinity for human IgG, as measured by its ability to inhibit rosette formation by IgG-coated RBCs.

The descriptions of the foregoing embodiments of the invention have been presented for purpose of illustration and description. They are not intended to be exhaustive or to limit the invention to the precise forms disclosed, and obviously many modifications and variations are possible in light of the above teaching. The embodiments were chosen and described in order to best explain the principles of the invention and its practical application to thereby enable others skilled in the art to best utilize the invention in various embodiments and with various modifications as are suited to the particular use contemplated. It is intended that the scope of the invention be defined by the claims appended hereto.

Applicants have deposited pcD-hFcγR-16.2 with the American Type Culture Collection, Rockville, MD, USA (ATCC), under accession number 67565. This deposit was made under conditions as provided under ATCC's agreement for Culture Deposit for Patent Purposes, which assures that the deposit will be made available to the US Commissioner of Patents and Trademarks pursuant to 35 U.S.C. 122 and 37 C.F.R. 1.14, and will be made available to the public upon issue of a U.S. patent, which requires that the deposit be maintained. Availability of the deposited strain is not to be construed as a license to practise the invention in contravention of the rights granted under the authority of any government in accordance with its patent laws.

**Claims**

1. A nucleic acid capable of encoding a polypeptide capable of binding the Fc portion of human immunoglobulin G, the polypeptide being defined by the formula:

Ala - Ala - Ala - Pro - Pro - Lys - Ala - Val - Leu --
Lys
-Leu - Glu - Pro - Pro - Trp - Ile - Asn - Val - Leu-
- Gln
-Glu - Asp - Ser - Val - Thr - Leu - Thr - Cys - Gln - Gly
-Ala - Arg - Ser - Pro - Glu - Ser - Asp - Ser - Ile-
- Gln
-Trp - Phe - His - Asn - Gly - Asn - Leu - Ile - Pro - Thr
-His - Thr - Gln - Pro - Ser - Tyr - Arg - Phe - Lys - Ala
-Asn - Asn - Asn - Asp - Ser - Gly - Glu - Tyr - -
Thr - Cys
-Gln - Thr - Gly - Gln - Thr - Ser - Leu - Ser - Asp - Pro
-Val - His - Leu - Thr - Val - Leu - Ser - Glu - Trp-
- Leu
-Val - Leu - Gln - Thr - Pro - His - Leu - Glu - Phe - Gln
-Glu - Gly - Glu - Thr - Ile - Met - Leu - Arg - Cys - His
-Ser - Trp - Lys - Asp - Lys - Pro - Leu - Val - Lys - Val
-Thr - Phe - Phe - Gln - Asn - Gly - Lys - Ser - -
Gln - Lys
-Phe - Ser - Arg - Leu - Asp - Pro - Thr - Phe - -
Ser - Ile
-Pro - Gln - Ala - Asn - His - Ser - His - Ser - Gly - Asp
-Tyr - His - Cys - Thr - Gly - Asn - Ile - Gly - Tyr --
Thr
-Leu - Phe - Ser - Ser - Lys - Pro - Val - Thr - Ile - Thr-
Val - Gln - Val - Pro - Ser - Met - Gly - Ser.

2. The nucleic acid of claim 1 wherein said polypeptide is defined by the formula:

Ala - Ala - Ala - Pro - Pro - Lys - Ala - Val - Leu --
Lys
-Leu - Glu - Pro - Pro - Trp - Ile - Asn - Val - Leu-
- Gln
-Glu - Asp - Ser - Val - Thr - Leu - Thr - Cys - Gln - Gly
-Ala - Arg - Ser - Pro - Glu - Ser - Asp - Ser - Ile-
- Gln
-Trp - Phe - His - Asn - Gly - Asn - Leu - Ile - Pro - Thr
-His - Thr - Gln - Pro - Ser - Tyr - Arg - Phe - Lys - Ala
-Asn - Asn - Asn - Asp - Ser - Gly - Glu - Tyr - -
Thr - Cys
-Gln - Thr - Gly - Gln - Thr - Ser - Leu - Ser - Asp - Pro
-Val - His - Leu - Thr - Val - Leu - Ser - Glu - Trp-
- Leu
-Val - Leu - Gln - Thr - Pro - His - Leu - Glu - Phe - Gln
-Glu - Gly - Glu - Thr - Ile - Met - Leu - Arg - Cys - His
-Ser - Trp - Lys - Asp - Lys - Pro - Leu - Val - Lys - Val
-Thr - Phe - Phe - Gln - Asn - Gly - Lys - Ser - -

Gln - Lys
-Phe - Ser - Arg - Leu - Asp - Pro - Thr - Phe - -
Ser - Ile
-Pro - Gln - Ala - Asn - His - Ser - His - Ser - Gly - Asp
-Tyr - His - Cys - Thr - Gly - Asn - Ile - Gly - Tyr --
Thr
-Leu - Phe - Ser - Ser - Lys - Pro - Val - Thr - Ile - Thr
-Val - Gln - Val - Pro - Ser - Met - Gly - Ser - Ser-
- Ser
-Pro - Met - Gly - Ile - Ile - Val - Ala - Val - Val - -
Ile
-Ala - Thr - Ala - Val - Ala - Ala - Ile - Val - Ala - -
Ala
-Val - Val - Ala - Leu - Ile - Tyr - Cys - Arg - Lys --
Lys
-Arg - Ile - Ser - Ala - Asn - Ser - Thr - Asp - Pro - Val
-Lys - Ala - Ala - Gln - Phe - Glu - Pro - Pro - Gly - Arg
-Gln - Met - Ile - Ala - Ile - Arg - Lys - Arg - Gln -
- Leu -
Glu - Glu - Thr - Asn - Asn - Asp - Tyr - Glu - Thr - Ala
-Asp - Gly - Gly - Tyr - Met - Thr - Leu - Asn - Pro - Arg
-Ala - Pro - Thr - Asp - Asp - Asp - Lys - Asn - -
Ile - Tyr
-Leu - Thr - Leu - Pro - Pro - Asn - Asp - His - -
Val - Asn -Ser - Asn - Asn.

3. A glycosylated or unglycosylated polypeptide capable of binding the Fc portion of human immunoglobulin G, the polypeptide comprising the following amino acid sequence:

Ala - Ala - Ala - Pro - Pro - Lys - Ala - Val - Leu --
Lys
-Leu - Glu - Pro - Pro - Trp - Ile - Asn - Val - Leu-
- Gln
-Glu - Asp - Ser - Val - Thr - Leu - Thr - Cys - Gln - Gly
-Ala - Arg - Ser - Pro - Glu - Ser - Asp - Ser - Ile-
- Gln
-Trp - Phe - His - Asn - Gly - Asn - Leu - Ile - Pro - Thr
-His - Thr - Gln - Pro - Ser - Tyr - Arg - Phe - Lys - Ala
-Asn - Asn - Asn - Asp - Ser - Gly - Glu - Tyr - -
Thr - Cys
-Gln - Thr - Gly - Gln - Thr - Ser - Leu - Ser - Asp - Pro
-Val - His - Leu - Thr - Val - Leu - Ser - Glu - Trp-
- Leu
-Val - Leu - Gln - Thr - Pro - His - Leu - Glu - Phe - Gln
-Glu - Gly - Glu - Thr - Ile - Met - Leu - Arg - Cys - His
-Ser - Trp - Lys - Asp - Lys - Pro - Leu - Val - Lys - Val
-Thr - Phe - Phe - Gln - Asn - Gly - Lys - Ser - -
Gln - Lys
-Phe - Ser - Arg - Leu - Asp - Pro - Thr - Phe - -
Ser - Ile
-Pro - Gln - Ala - Asn - His - Ser - His - Ser - Gly - Asp

-Tyr - His - Cys - Thr - Gly - Asn - Ile - Gly - Tyr -- Thr -
Leu - Phe - Ser - Ser - Lys - Pro - Val - Thr - Ile -- Thr -Val - Gln - Val - Pro - Ser - Met - Gly - Ser.

4. A method of detecting immune complexes in a sample, the method comprising the steps of:

providing cells transformed by a vector carrying a cDNA insert encoding an Fcγ receptor, the Fcγ receptor being expressed on the surface of the cells;

exposing the cells to the sample containing immune complex so that the immune complex can adhere to the surface of the cells by way of the Fcγ receptors;

labeling the immune complex adhering to the surfaces of the cells; and

measuring the amount of labeled immune complex adhering to the cells.

5. The method of claim 4 wherein said cells are mouse L cells and said vector is pcD-hFcγR-16.2.

**FIGURE 1**

EP 0 319 307 A2

```
         10                      ·34·                    49                     64                    ··· 79                    · 94
GGGGGGGGGAC AGTGCTGGG ATG ACT ATG GAG ACC CAA ATG TCT CAG AAT GTA TGT CCC AGA AAC CTG TGG CTG CTT CAA CCA TTG ACA GTT TTG CTG CTG CTG
                     MET Thr MET Glu Thr Gln MET Ser Gln Asn Val Cys Pro Arg Asn Leu Trp Leu Leu Gln Pro Leu Thr Val Leu Leu Leu Leu

         109                     +1                     139                    154                    169                       184                     199
GCT TCT GCA GAC AGT CAA GCT GCA GCT CCC CCA AAG GCT GTG CTG AAA CTT GAG CCC CCG TGG ATC AAC GTG CTC CAG GAG GAC TCT GTG ACT CTG ACA
Ala Ser Ala Asp Ser Gln Ala Ala Ala Pro Pro Lys Ala Val Leu Lys Leu Glu Pro Pro Trp Ile Asn Val Leu Gln Glu Asp Ser Val Thr Leu Thr

               214        1        229                   244                    259                    274                     289
TGC CAG GGG GCT CGC AGC CCT GAG AGC GAC TCC ATT CAG TGG TTC CAC AAT GGG AAT CTC ATT CCC ACC CAC ACG CAG CCC AGC TAC AGG TTC AAG GCC
Cys Gln Gly Ala Arg Ser Pro Glu Ser Asp Ser Ile Gln Trp Phe His Asn Gly Asn Leu Ile Pro Thr His Thr Gln Pro Ser Tyr Arg Phe Lys Ala

      304                     319                    334                     349                    364                       379                     394
AAC AAC AAT GAC AGC GGG GAG TAC ACG TGC CAG ACT GGC CAG ACC AGC CTC AGC GAC CCT GTG CAT CTG ACT GTG CTT TCC GAA TGG CTG GTG CTC CAG
Asn Asn Asn Asp Ser Gly Glu Tyr Thr Cys Gln Thr Gly Gln Thr Ser Leu Ser Asp Pro Val His Leu Thr Val Leu Ser Glu Trp Leu Val Leu Gln

         409                     424                    439                    454        2       469                      484                     499
ACC CCT CAC CTG GAG TTC CAG GAG GGA GAA ACC ATC ATG CTG AGG TGC CAC AGC TGG AAG GAC AAG CCT CTG GTC AAG GTC ACA TTC TTC CAG AAT GGA
Thr Pro His Leu Glu Phe Gln Glu Gly Glu Thr Ile MET Leu Arg Cys His Ser Trp Lys Asp Lys Pro Leu Val Lys Val Thr Phe Phe Gln Asn Gly

            514                    529                    544                    559                    574                     589
AAA TCC CAG AAA TTC TCC CGT TTG GAT CCC ACC TTC TCC ATC CCA CAA GCA AAC CAC AGT CAC AGT GGT GAT TAC CAC TGC ACA GGA AAC ATA GGC TAC
Lys Ser Gln Lys Phe Ser Arg Leu Asp Pro Thr Phe Ser Ile Pro Gln Ala Asn His Ser His Ser Gly Asp Tyr His Cys Thr Gly Asn Ile Gly Tyr

      604                     619                    634                     649                    664                       679                     694
ACG CTG TTC TCA TCC AAG CCT GTG ACC ATC ACT GTC CAA GTG CCC AGC ATG GGC AGC TCT TCA CCA ATG GGG ATC ATT GTG GCT GTG GTC ATT GCG ACT
Thr Leu Phe Ser Ser Lys Pro Val Thr Ile Thr Val Gln Val Pro Ser MET Gly Ser Ser Ser Pro MET Gly Ile Ile Val Ala Val Val Ile Ala Thr

               709                    724                    739                    754                    769                     784
GCT GTA GCA GCG ATT GTT GCT GCT GTA GTG GCC TTG ATC TAC TGC AGG AAA AAG CGG ATT TCA GCC AAT TCC ACT GAT CCT GTG AAG GCT GCC CAA TTT
Ala Val Ala Ala Ile Val Ala Ala Val Val Ala Leu Ile Tyr Cys Arg Lys Lys Arg Ile Ser Ala Asn Ser Thr Asp Pro Val Lys Ala Ala Gln Phe

      799                     814                    829                     844                    859                       874                     889
GAG CCA CCT GGA CGT CAA ATG ATT GCC ATC AGA AAG AGA CAA CTT GAA GAA ACC AAC AAT GAC TAT GAA ACA GCT GAC GGC GGC TAC ATG ACT CTG AAC
Glu Pro Pro Gly Arg Gln MET Ile Ala Ile Arg Lys Arg Gln Leu Glu Glu Thr Asn Asn Asp Tyr Glu Thr Ala Asp Gly Gly Tyr MET Thr Leu Asn

         904                     919                    934                    949                    964                     983         993
CCC AGG GCA CCT ACT GAC GAT GAT AAA AAC ATC TAC CTG ACT CTT CCT CCC AAC GAC CAT GTC AAC AGT AAT AAC TAA AGAGTAACGT TATGCCATGT
Pro Arg Ala Pro Thr Asp Asp Asp Lys Asn Ile Tyr Leu Thr Leu Pro Pro Asn Asp His Val Asn Ser Asn Asn  . ·

   1003       1013        1023       1033       1043       1053       1063       1073        1083       1093        1103       1113
GGTCATACTC TCAGCTTGCG TATGGATGCA AAAAAGAGGG GAATTGTTAA AGGAAAATTT AAATGGAGAC TGGAAAAATC CTGAGCAAAC AAAACCACCT GGCCCTTAGA AATAGCTTTA

   1123       1133       1143       1153       1163       1173       1183       1193       1203       1213       1223       1233
ACTTTGCTTA AACTACAAAC ACAAGCAAAA CTTCACGGGG TCATACTACA TACAAGCATA AGCAAAACTT AACTTGGATC ATTTCTGGTA AATGCTTATG TTAGAAATAA GACAACCCCA

   1243       1253       1263       1273       1283       1293       1303       1313       1323       1333       1343       1353
GCCAATCACA AGCAGCCTAC TAACATATAA TTAGGTGACT AGGGACTTTC TAAGAAGATA CCTACCCCCA AAAAACAATT ATGTAATTGA AAACCAACCG ATTGCCTTTA TTTTGCTTCC

   1363       1373       1383       1393       1403
ACATTTTCCC AATAAATACT TGCCTGTGAC ATTTTGCCAC TGGAACACTA (A)n
```

**FIGURE 2**